# EUROPEAN PATENT SPECIFICATION

(11) **EP 1 948 071 B1**
(45) Date of publication and mention of the grant of the patent: **07.10.2015**
(21) Application number: 06837766.2
(22) Date of filing: 16.11.2006
(51) Int. Cl.: A61F 2/01, A61F 2/82

(54) **BIODEGRADABLE VASCULAR FILTER**
BIOABBAUBARER GEFÄSSFILTER
FILTRE VASCULAIRE BIODÉGRADABLE

(30) Priority: 17.11.2005 US 281128
(43) Date of publication of application: 30.07.2008
(73) Proprietor: Novate Medical Ltd., Dublin 2 (IE)
(72) Inventor: SOSNOWSKI, Stephen A., Vista, CA 92084 (US); ROSENTHAL, David, Marrietta, GA 30067 (US)
(74) Representative: Weldon, Michael James
(86) International application number: PCT/US2006/044482
(87) International publication number: WO 2007/061743

(56) References cited:
- WO-A-2006/020425
- WO-A2-02/22048
- US-A1- 2003 176 888

## Description

### BACKGROUND OF THE DISCLOSURE

The present disclosure relates to novel enhanced surgical tools. In particular, the present disclosure describes apparatus useful for vascular surgical and interventional radiological procedures having needs for improved trapping surfaces.

The documented needs, for example, relative to vena caval filters have driven the development of new devices to prevent migration of thrombus to the lungs. Providing larger and more efficient trapping surfaces while minimizing insertion issues is a longstanding need in the art, and the advent of retrievable IVC filters merely underscores the need without addressing the issues which plague current treatment modes and modalities.

When surgical or radiological interventions are done, and when patients present with conditions or disease etiologies that relate to the generation of blood clots, or thrombus, medical devices have been introduced which function to prevent these from passing into other areas of the body where they can be harmful or lethal.

Exemplary devices which have been used to manage such conditions have generated a plurality of longstanding needs yet to be addressed. Incorporated by reference herein, and illustrative of these predicate devices having generated most of these shortcomings are found in the following United States Letters Patents, which served to define the state of the art prior to the advent of the instant teachings:
5,375,612; 6,267,776; 6,932,832; 6,669,721; 6,666,882; 6,652,558; 6,582,447; 6,669,721; 6,605,111; 6,517,559; and 6,267,776. WO02/22048 discloses a vascular system according to the preamble of claim 1.

Each of these references has been studied, as have the devices that embody them, as discussed below, and found to be differentiated from the subject matter of the present invention. Further, it is noted that within the last five years, seventy-nine (79) devices have been taken off of the market because of the potential for morbidity as a side effect. Likewise, carotid stenting itself has been questioned, and the need for filters has only increased.

For these reasons and because of the urgent needs to provide for treatments for patients that work better than the state of the art, the instant disclosure is hereby offered for consideration.

### SUMMARY OF THE DISCLOSURE

The present inventors have overcome longstanding issues in preventing recurrent pulmonary embolism, among other things, by percutaneous placement of an improved biodegradable filter in the vena cava. This enhanced treatment modality addresses pulmonary thromboembolism when anticoagulants are contraindicated, treats thromboembolic disease, addresses massive pulmonary embolism and chronic, recurrent embolisms better than existing devices. Safer devices are sorely needed, along with those that are effective.

According to a feature of the present invention there is provided a biodegradable vascular filter system, which comprises, in combination, a self-expanding apparatus which undergoes a phase change enabling it to move from a first position to a second position, operatively connected with a plurality of polymeric string-like members, which members expand from a slackened to a tensioned state in conjunction with the phase change of the associated apparatus, wherein the system when in at least one vessel and/or lumen is effective for trapping matter traveling therethrough.

Other aspects of the invention are set out in claims 2 to 10.

According to another feature of the disclosure a process for mitigating insult and injury by thrombus comprising, in combination, providing a vascular filter device further comprising a nitinol skeleton operatively linked to a biodegradable polymer, implanting the vascular filter device at a desired location within the vessel and leaving the vascular filter device in situ.

According to a feature of the disclosure there is provided a biodegradable vascular filter system, which comprises, in combination, apparatus effective for being lodged within a vessel and a plurality of polymeric string-like members, which members may be disposed in at least one configuration from a slackened to a tensioned state when implanted within the vessel, including at least a conical and a frusto-conical configuration, wherein the system when implanted in at least one of a vessel and a body lumen and is effective for trapping thrombi traveling therethrough.

Briefly stated, vascular systems of the invention for trapping emboli utilize self-expanding skeletons and biodegradable polymer systems, for example stent-like Nitinol® elements and PLGA, to address longstanding issues related to thrombus capture without deleterious impacts on the vasculature or other negative artifacts of the procedure by at least partial post-use dissolution in situ.

### BRIEF DESCRIPTION OF THE DRAWINGS

The above-mentioned features and objects of the present disclosure will become more apparent with reference to the following description taken in conjunction with the accompanying drawings wherein like reference numerals denote like elements and in which:
Figure 1 is a schematic showing a biodegradable filter according to embodiments of the present disclosure;
Figure 2 is a schematic top view showing a biodegradable filter according to embodiments of the present disclosure;
Figure 3 is a schematic partial perspective view of a biodegradable filter according to embodiment of the present disclosure;
Figure 3A is another schematic showing a deployment in which a first a-tensional state is depicted;
Figure 3B is an additional schematic showing a deployment in which a second a-tensional state is depicted;
Figure 4 is a schematic side view of a biodegradable filter according to embodiments of the present disclosure;
Figure 5 is a schematic side view according to embodiments of the present disclosure;
Figure 6 is a partial plan view of a biodegradable filter according to embodiments of the present disclosure;
Figure 7 is a side view of a biodegradable filter according to embodiments of the present disclosure; and
Figure 8 is a side view of a biodegradable filter.

### DETAILED DESCRIPTION OF PREFERRED EMBODIMENTS OF THE DISCLOSURE

The present inventors have discovered a novel enhanced process and products to mitigate thrombic insult, injury and related and attendant harms. By combining a shape memory alloy, or plastic, for example Nitinol®, and biodegradable polymer systems an improved surgical filter effective to arrest transmissions of thrombus is disclosed. Processes using various embodiments are also taught, where the polymer systems are absorbed, or degrade while the skeleton grows into the vessel.

Those skilled in the art readily understand that a biodegradable polymer system includes any related biocompatible set of moieties approved, or to be approved, for use in animals. By way of illustrative example, poly(lactic-co-glycolic) acid (hereafter "PLGA"), is readily substitutable for any number of biodegradable polymers having a strong history of usage in U.S. Food and Drug Administration ("FDA") approved devices.

Likewise, delivery systems are conventional, and used by all of the major cardiovascular disease companies, which must be given consideration in the design and execution of such medical devices. The trend in these devices is procurement of larger and more effective trapping surfaces and smaller and less invasive insertion systems.

Prominent examples of the other devices in these fields include the LP brand of filter from B. Braun, the Gunther Tulip™ brand of Vena Cava filter, and the Cordis Optease brand of permanent vena cava filter, in addition to the Recovery brand of filter system offered by Bard Peripheral Vascular, a division of C.R. Bard Incorporated. Unacceptably high records of adverse events are associated with all of these devices.

PLGA may be synthesized by the co-polymerization of glycolide and lactide. The present inventors have searched predicate devices and approaches but are unaware of other usages of PLGA or other such biodegradable polymer such as those taught according to the present disclosure. However, those skilled in the art readily understand that any number of materials with an *in situ* history shall suffice for the purposes of the present invention.

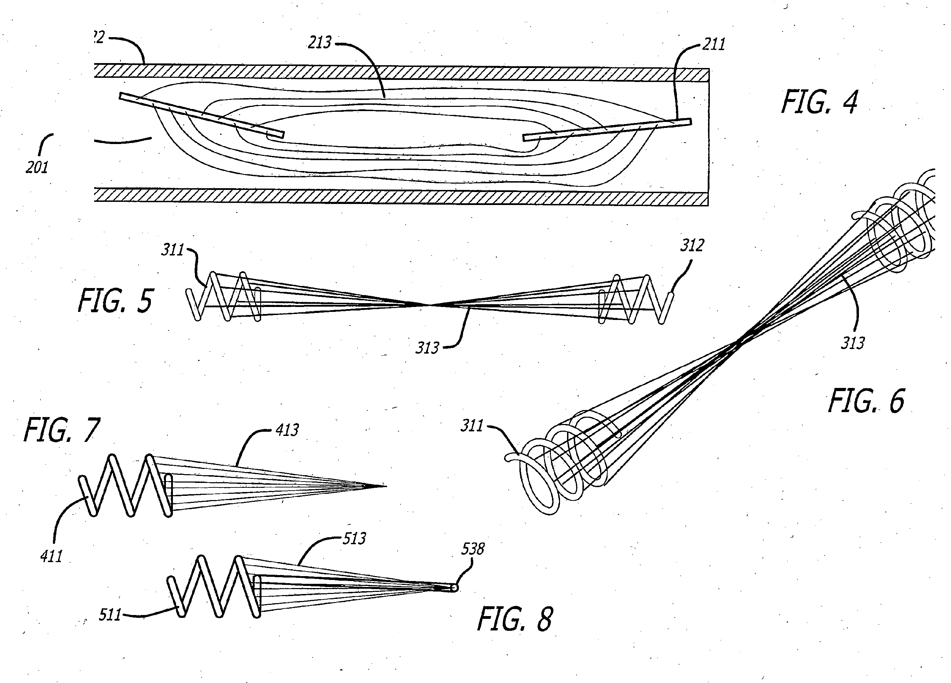

Likewise, although indications are clearly available for improved filters nothing which has effectively addressed and solved the problems at which the present invention is directed currently is known. By way of further example of the need for the present invention vascular filters have commonly been adapted or used in a other lumens as needed. See, for example Fig. 5 through Fig. 8.

Another known filter is the Greenfield® brand of filter from Boston Scientific®. Each of these devices have been studied and found subject to various complications stemming from common challenges. The present disclosure overcomes such issues.

Filter occlusion, from trapped emboli, often results in adverse events ranging from renal failure, the need for heightened thrombolytic therapy, to death of the subject patients. Metal fatigue and fracture, poor flow characteristics and areas of stagnation also generate significant issues. Fixation hooks associated with known devices, and the high radial force associated with the deployment of known systems have also added vasculature insult and injury to the list.

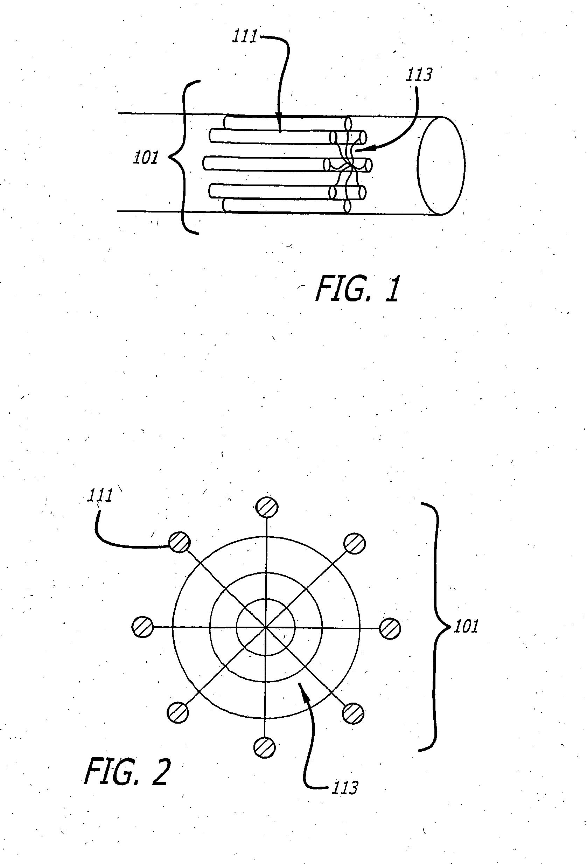

Turning now to Figure 1, novel enhanced biodegradable filter is generally and schematically illustrated as device 101 struts 111, as deployed leverage off of the benefits of Nitinol®, or "spring steel" which moves from a first (compacted) position to a second (expanded) condition upon release within an environment having a higher temperature - such as the desired lumen of a vessel. Artisans readily understand restraints may delay this expansion as commonly practiced within the catheter arts. PLGA matrix 113 provides for a trapping mechanism when tensioned by the expansion of Nitinol® struts 111 as the 'spring steel' moves from a first to a second position.

Referring now also to Figure 2, it is shown how expanded struts 111 of device 101 can render PLGA (or any other biodegradable polymer system, as set forth and discussed above and claimed below) matrix 113 effective to trap emboli, without the constraint of concomitant flow restriction. Deployment of device 101 does not cause vessel damage through high radial force, nor do damages by ripping into the vessel wall. Rather Nitinol® struts 111 merge gently with vessel walls, growing into the neointima of the vessels.

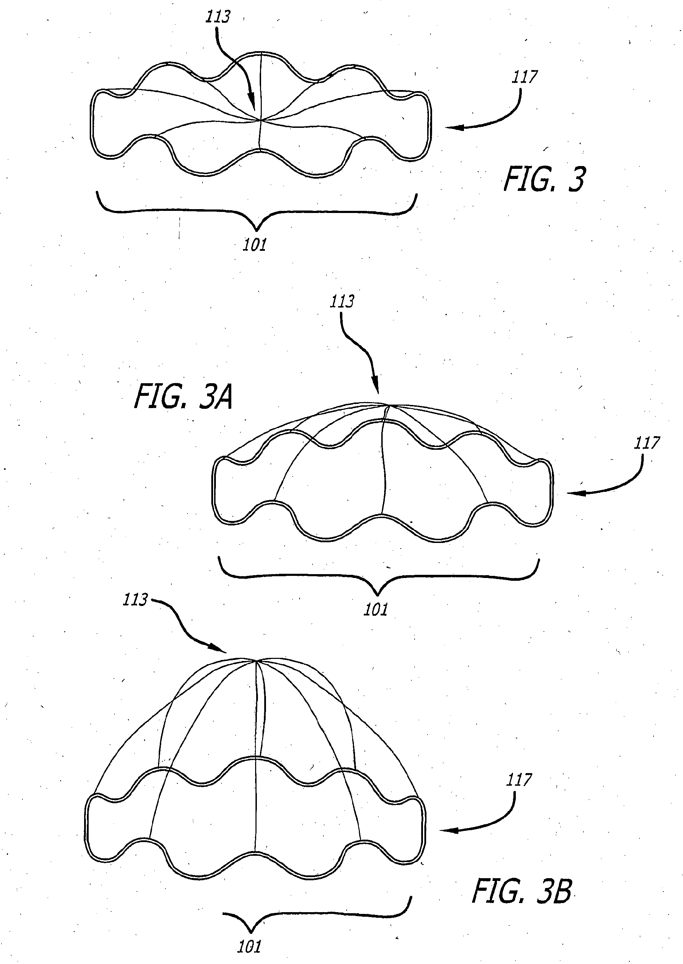

Turning now to Figure 3, a stent-like embodiment of device 100 features Nitinol® hoops 117, whose memory allows them to be situated within a delivery catheter and through minimally invasive techniques, delivered to an appropriate site. Those skilled in the art readily understand that the slackened view shown in this figure is often how devices according to instant teachings are most effectively deployed. Likewise, PLGA or other biodegradable bioabsorbable material may be used for hoops 117.

Figures 3A and 3B show how different degrees of tension may be employed in various different states, depending on where the instant system is used. Those skilled in the art readily understand where and how this best works, in terms of the human vasculature.

The medical device usage of shape-memory alloys, whose function as is well know to those skilled in the art in accordance with the SMART®-type of self expanding stent (Cordis Endovascular, Johnson & Johnson), to render device 101 effective to be delivered by known systems of catheters, arid to be placed at an appropriate juncture in a vessel without damaging the same. For example, placement in any known vessel by a femoral insertion of an introducer and guidewire system (available from Medtronic® AVE, Guidant®, Edward LifeSciences® LLC or Cook Endovascular as approved by the U.S. FDA), is conventional.

The benefits of stent-like device 101 , with for example PLGA web 113, are significant in comparison to known teachings. For example, as opposed to leaving the filter in the patient, or attempting to retrieve the same by dragging it out, each of which does more harm than good - the instant disclosure teaches leaving the device in, the PLGA to dissolve over time, while the remaining assembly is endothelialized and encased in the wall of the vessel.

Turning now to Figure 4, an alternate device is disclosed which has closer analogy to the Cook Endovascular Bird-Nest® brand of device. This biodegradable filter 201, once again is comprised of Nitinol® struts 211, which are shown in a first (compacted) condition within catheter/delivery system 222. Once more, "spring-steel" may be chilled, cooled or otherwise restrained to maintain this first state. Nitinol® 211 undergoes a phase change from austenite to martenite upon a correct temperature change and the "memory" it has allows the health-care provider to size it appropriately for the desired vessel. PLGA is an effective polymer system, and those skilled in the art will understand that others may be used as well.

Figures 5-8 disclose a second or expanded state of Nitinol® struts, respectively proximate 311 and distal 312 (411, 511) as used to filter emboli in different vessels. Figure 5, for example may be used for pregnant patients with thromboembolism. In such a disease state, extensive iliofemoral deep vein thrombosis with thrombolytic therapy or surgical thrombus-generating procedures are a major area of concern.

The disclosed devices in the field are designed to trap emboli during these procedures, but generally add more risk factors than they prevent. The instant disclosure overcomes these issues and allows surgeons and interventionalists an option.

It is also prominent in the literature that permanent vena cava filters often cause pulmonary embolisms, and other significant complications many of which are addressed and overcome by the instant teachings.

Figures 6-8 show customized versions which may be used as temporary filters. The present invention allows medical practitioners to address trapping issues at various locations in the vasculature, as known to artisans.

Likewise, Figure 7 and Figure 8 use Nitinol® stent-like members 411, 511 for trauma and orthopedic surgery with PLGA (and the like polymers) 413, 513 being custom-tailored also for pediatric, hepatic, biliary usage. Anchor 538 may also be used for smaller vessels or specialized approach where lumens are challenging to access or require alternate positioning means. Such usages are within the ambition of surgeons or interventional radiologists of skill in the art, and so further discussion is omitted at this time.

While the apparatus and method have been described in terms of what are presently considered to be the most practical and preferred embodiments, it is to be understood that the disclosure need not be limited to the disclosed embodiments. It is intended to cover various modifications and similar arrangements included within the scope of the claims, the scope of which should be accorded the broadest interpretation so as to encompass all such modifications and similar structures. The present disclosure includes any and all embodiments of the following claims.

## Claims

1. A vascular system (101) comprising:
a self-expanding apparatus (111) which undergoes a phase change enabling it to move from at least a compacted position to an expanded position, and
a filter (113) effective for, when implanted in at least one of a vessel and a body lumen, trapping thrombi;
**characterised in that**,
the filter (113) comprises a plurality of degradable polymeric string-like members, which members expand from a slackened to a tensioned state in conjunction with the phase change of the apparatus from the compacted position to the expanded position.

2. A vascular system as claimed in claim1, further comprising a supplemental restraining mechanism for maintaining the self-expanding apparatus in the compacted position until a desired release time.

3. A vascular system as claimed in either of claims 1 or 2, wherein the apparatus (111) comprises at least one shape memory alloy selected from the group consisting of Nitinol™ and other biocompatible metals.

4. A vascular system as claimed in claim 3, wherein the apparatus (111) comprises Nitinol™ struts and a plurality of string-like members comprised of PLGA.

5. A vascular system as claimed in any preceding claim, wherein the apparatus comprises at least one hoop (117) whose memory allows them to be situated within a delivery catheter and, through minimally invasive techniques, delivered to an appropriate site.

6. A vascular system as claimed in either of claims 1, 2, or 5, wherein the apparatus (111) comprises at least one polymer system selected from the group consisting of PLGA and other polymers which degrade at a pre-determined time.

7. A vascular system as claimed in any preceding claim, further comprising at least one drug eluting element.

8. A vascular system as claimed in any preceding claim, further comprising at least one of a bioabsorbable and a bioresorbable material.

9. A vascular system as claimed in any preceding claim, wherein the filter is retrievable.

10. A vascular system as claimed in any preceding claim, wherein the filter remains operative within the body during a critical period and degrades after the critical period.

## Patentansprüche

1. Gefäßsystem (101), das Folgendes umfasst:
eine selbstexpandierende Vorrichtung (111), die eine Phasenänderung durchläuft, was ermöglicht, dass sie sich von mindestens einer komprimierten Position zu einer expandierten Position bewegt, und
einen Filter (113), der bei Implantation in mindestens einem von einem Gefäß und einem Körperlumen das Abfangen von Thromben bewirkt;
**dadurch gekennzeichnet, dass**
der Filter (113) eine Vielzahl an abbaubaren polymeren schnurartigen Elementen umfasst, welche Elemente von einem schlaffen zu einem gespannten Zustand in Verbindung mit der Phasenänderung der Vorrichtung von der komprimierten Position zur expandierten Position expandieren.

2. Gefäßsystem nach Anspruch 1, das weiter einen ergänzenden einschränkenden Mechanismus zur Aufrechterhaltung der selbstexpandierenden Vorrichtung in der komprimierten Position bis zu einer gewünschten Freigabezeit umfasst.

3. Gefäßsystem nach einem der Ansprüche 1 oder 2, worin die Vorrichtung (111) mindestens eine Formgedächtnislegierung umfasst, die aus der Gruppe ausgewählt ist, die aus Nitinol™ und anderen biokompatiblen Metallen besteht.

4. Gefäßsystem nach Anspruch 3, worin die Vorrichtung (111) Nitinol™-Streben und eine Vielzahl an schnurartigen Elementen, die aus PLGA bestehen, umfasst.

5. Gefäßsystem nach einem vorstehenden Anspruch, worin die Vorrichtung mindestens einen Reifen (117) umfasst, dessen Gedächtnis ermöglicht, dass sie innerhalb eines Einführkatheters eingebracht werden und, durch minimal invasive Techniken, an die entsprechende Stelle abgegeben werden.

6. Gefäßsystem nach einem der Ansprüche 1, 2 oder 5, worin die Vorrichtung (111) mindestens ein Polymersystem umfasst, das aus der Gruppe ausgewählt ist, die aus PLGA und anderen Polymeren besteht, die in einer vorbestimmten Zeit abgebaut werden.

7. Gefäßsystem nach einem vorstehenden Anspruch, das weiter mindestens ein Medikamenten-freisetzendes Element umfasst.

8. Gefäßsystem nach einem vorstehenden Anspruch, das weiter mindestens eines von einem bioabsorbierbaren und einem bioresorbierbaren Material umfasst.

9. Gefäßsystem nach einem vorstehenden Anspruch, worin der Filter rückführbar ist.

10. Gefäßsystem nach einem vorstehenden Anspruch, worin der Filter im Körper während eines kritischen Zeitraums funktionsfähig bleibt und nach dem kritischen Zeitraum abgebaut wird.

## Revendications

1. Système vasculaire (101) comprenant :
un dispositif auto-extensible (111) qui subit un changement de phase lui permettant de passer d'au moins une position compactée à une position déployée et
un filtre (113) qui, une fois implanté dans un vaisseau et/ou lumière corporelle, est capable de piéger des thrombi ;
**caractérisé en ce que**
le filtre (113) comprend une pluralité de membres filiformes polymères dégradables, lesdits membres passant d'un état relâché à un état tendu lorsque le dispositif subit un changement de phase et passe d'une position compactée à une position déployée.

2. Système vasculaire selon la revendication 1, qui comprend en outre un mécanisme de retenue supplémentaire pour maintenir le dispositif auto-extensible en position compactée jusqu'au moment où son déploiement est souhaité.

3. Système vasculaire selon l'une ou l'autre des revendications 1 ou 2, dans lequel le dispositif (111) comprend au moins un alliage à mémoire de forme sélectionné dans le groupe consistent en le Nitinol™ et d'autres métaux biocompatibles.

4. Système vasculaire selon la revendication 3, dans lequel le dispositif (111) comprend des spires en Nitinol™ et une pluralité de membres filiformes composés de PLGA.

5. Système vasculaire selon l'une quelconque des revendications précédentes, dans lequel le dispositif comprend au moins un arceau (117) doté d'une mémoire qui lui permet d'être inséré dans un cathéter de pose et délivré en un site approprié par le biais de techniques au minimum invasives.

6. Système vasculaire selon l'une quelconque des revendications 1,2 ou 5, dans lequel le dispositif (111) comprend au moins un système polymère sélectionné dans le groupe consistant en le PLGA et d'autres polymères qui sont dégradés après une période de temps prédéterminée.

7. Système vasculaire selon l'une quelconque des revendications précédentes, qui comprend en outre au moins un élément à élution médicamenteuse.

8. Système vasculaire selon l'une quelconque des revendications précédentes, qui comprend en outre un matériau bioabsorbable et/ou biorésorbable.

9. Système vasculaire selon l'une quelconque des revendications précédentes, dans lequel le filtre est récupérable.

10. Système vasculaire selon l'une quelconque des revendications précédentes, dans lequel le filtre demeure opérationnel dans l'organisme pendant une période critique et est dégradé après la période critique.
